**Europäisches Patentamt**

**European Patent Office**

**Office européen des brevets**

(19)

(11) Publication number: **0 184 041**
**A1**

(12) # EUROPEAN PATENT APPLICATION

(21) Application number: **85114433.7**

(22) Date of filing: **13.11.85**

(51) Int. Cl.⁴: **C 07 D 221/28**

(30) Priority: **13.11.84 US 670043**

(43) Date of publication of application:
**11.06.86 Bulletin 86/24**

(84) Designated Contracting States:
**AT BE CH DE FR GB IT LI NL SE**

(71) Applicant: **MILES LABORATORIES, INC.**
**1127 Myrtle Street**
**Elkhart Indiana 46514(US)**

(72) Inventor: **Reverman, Lawrence Francis**
**59387 County Road 9**
**South Elkhart Indiana 46517(US)**

(74) Representative: **Jesse, Ralf-Rüdiger, Dr. et al,**
**Bayer AG Konzernverwaltung RP Patentabteilung**
**D-5090 Leverkusen 1 Bayerwerk(DE)**

(54) 6-Methylene morphinan purification technique.

(57) A method for the purification/recovery of the analgesic/ narcotic antagonist 17-cyclobutylmethyl-3-hydroxy-8ß-methyl-6-methylene morphinane is disclosed. Said method avoids chromatographic purification techniques and provides a practical method for the purification of large quantities of said compound.

EP 0 184 041 A1

- 1 -

# 6-METHYLENE MORPHINAN PURIFICATION TECHNIQUE

The present invention is directed to a purification/recovery technique in the synthesis of 17-cyclobutylmethyl-3-hydroxy-8β-methyl-6-methylene morphinane. Said compound is a 6-methylene morphinan derivative exhibiting analgesic/narcotic antagonist properties.

## DESCRIPTION OF PERTINENT ART

17-cyclobutylmethyl-3-hydroxy-8β-methyl-6-methylene morphinane (hereinafter alternatively referred to as "TR-5379") is disclosed and claimed in U.S. Patent No. 4,259,329 issued March 31, 1981 to Kotick, et al. As described therein, said compound is prepared by the reaction of methylene-triphenylphosphorane with 17-cyclobutylmethyl-3-methoxy-8β-methyl-morphinan-6-one in a suitable inert solvent under an inert atmosphere. Purification of the resulting compound (i.e., TR-5379) is subsequently achieved by column chromatography using Silica Gel G. However, this purification

MS-1364

technique suffers from the disadvantage of being impractical for large scale production of said compound due to the large quantities of Silica Gel G and large volumes of organic solvents necessary to conduct said purification. The method described herein teaches a procedure whereby column chromatography is eliminated as a means of recovering TR-5379 thereby lessening the time spent for isolation and purification of said compound as well as providing a method for the purification of large quantities of TR-5379.

## SUMMARY OF THE INVENTION

The present invention discloses a method for recovering the compound 17-cyclobutylmethyl-3-hydroxy-8β-methyl-6-methylene morphinane (i.e., TR-5379) from an inert organic solvent extract which contains said compound. The method includes the steps of: (a) adding said inert organic solvent extract to a dilute hydrochloric acid solution thereby forming a crystalline, insoluble hydrochloride salt of said compound; (b) isolating said insoluble hydrochloride salt; (c) adding said insoluble hydrochloride salt to an alkaline aqueous medium thereby converting said salt to said compound; and (d) extracting said compound from said medium by contacting said medium with an organic solvent and recovering therefrom said compound.

MS-1364

DETAILED DESCRIPTION OF THE INVENTION

The following nonlimiting example describes the general preparation of TR-5379 and the recovery procedure of the present invention.

- A solution of methylenetriphenylphosphorane was prepared from sodium hydride (0.66 mole) and methyltriphenylphosphonium bromide (237.4 grams) in 900 milliliters (ml) of dimethylsulfoxide under an argon atmosphere. To this was added a solution of 75 grams (g) of 17-cyclobutylmethyl-3-hydroxy-8β-methyl-morphinane-6-one in 350 ml of dimethylsulfoxide. The resulting reaction mixture was stirred at 80° C for three hours and allowed to cool. The cooled mixture was diluted with ice and concentrated ammonium hydroxide and extracted three times with approximately 1 liter portions of toluene. The toluene extracts were combined and washed four times with about 500 ml portions of water, dried and subsequently added dropwise to a 5% (by weight) hydrochloric acid solution. The resulting insoluble hydrochloride salt was filtered, washed several times with toluene, and then re-slurried in about 500 ml of fresh toluene. The solid was again isolated by filtration and subsequently suspended in about 500 ml of·water (pH adjusted to about 10-11 with concentrated ammonium hydroxide) and the mixture extracted three times with about 500 ml portions of a mixture of

MS-1364

ether:toluene (1:1). The combined organic extracts were washed four times with about 500 ml portions of water, dried and evaporated to give 65 g of the desired 17-cyclobutylmethyl-3-hydroxy-8β-methyl-6-methylene morphinane as a tan foam.

IR and NMR spectral characteristics compared favorably to an authentic sample prepared as described in U.S. Patent No. 4,259,329.

By way of example, the technique of the instant invention was illustrated with the compound TR-5379. However, the skilled artisan will readily discern that this technique may have applicability in the preparation of other 6-methylene-morphinan compounds. Accordingly, contemplated equivalent compounds to which the purification/recovery techniques of the present invention may be applied include those compounds disclosed by Kotick et al in U.S. Patent No. 4,259,329.

MS-1364

0184041

WHAT IS CLAIMED IS:

1. A method for recovering the compound 17-cyclobutylmethyl-3-hydroxy-8β-methyl-6-methylene morphinane from an inert organic solvent extract containing said compound which comprises the steps of:

a) adding said inert organic solvent extract to a dilute hydrochloric acid solution thereby forming a crystalline, insoluble hydrochloride salt of said compound:

b) isolating said insoluble hydrochloride salt;

c) adding said insoluble hydrochloride salt to an alkaline aqueous medium thereby converting said salt to said compound; and

d) extracting said compound from said medium by contacting said medium with an organic solvent and recovering therefrom said compound.

2. The method of claim 1 wherein said inert organic solvent extract is a toluene extract.

3. The method of Claim 1 wherein the dilute hydrochloric acid concentration of step (a) is about 5 percent by weight.

4. The method of Claim 1 wherein said alkaline aqueous medium of step (c) has a pH of about 11.

MS-1364

5. The method of Claim 1 wherein the organic solvent of step (d) is a mixture of ether and toluene in a ratio of about 1:1.

MS-1364

## DOCUMENTS CONSIDERED TO BE RELEVANT

| Category | Citation of document with indication  where appropriate, of relevant passages | Relevant to claim | CLASSIFICATION OF THE APPLICATION (Int Cl 4) |
|---|---|---|---|
| A | EP-A-0 079 556  (F. HOFFMANN-LA ROCHE & CO. AG) <br> * page 28, lines 22-35 * | 1,4 | C 07 D 221/28 |
| A | US-A-3 803 150  (I. MONKOVIC et al.) <br> * example 10 * | 1 | |
| A | US-E-  30 452  (E. MOHACSI) <br> * column 16, lines 10-19 * | 1 | |
| A | GB-A-1 043 859  (F. HOFFMANN-LA ROCHE & CO. AG) <br> * page 4, lines 98-106 * | 1 | |
| A | EP-A-0 027 925  (MILES LABORATORIES INC.) <br> * example 4 * | | TECHNICAL FIELDS SEARCHED (Int. Cl 4) <br><br> C 07 D 221/28 |

The present search report has been drawn up for all claims

| Place of search | Date of completion of the search | Examiner |
|---|---|---|
| BERLIN | 04-02-1986 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82